# EUROPEAN PATENT SPECIFICATION

(11) **EP 3 011 957 B1**
(45) Date of publication and mention of the grant of the patent: **11.04.2018**
(21) Application number: 15185378.5
(22) Date of filing: 16.09.2015
(51) Int. Cl.: A61K 31/136, A61K 31/145, A61K 31/167, A61P 9/00, A61P 9/10, A61P 17/02, A61P 13/12, A61P 17/14, A61P 15/10

(54) **COMPOSITION FOR STIMULATING NEOVASCULARIZATION AT A SITE IN A SUBJECT COMPRISING DAPSONE AND USE THEREOF**
ZUSAMMENSETZUNG ZUR STIMULIERUNG DER NEOVASKULARISATION AN EINER STELLE IN EINEM PATIENTEN MIT DAPSON UND VERWENDUNG DAVON
COMPOSITION POUR STIMULER LA NÉOVASCULARISATION AU NIVEAU D'UN SITE CHEZ UN SUJET COMPRENANT DE LA DAPSONE ET SON UTILISATION

(30) Priority: 22.10.2014 KR 20140143603
(43) Date of publication of application: 27.04.2016
(73) Proprietor: Samsung Electronics Co., Ltd., Gyeonggi-do 443-742 (KR)
(72) Inventor: Koh, Youngjun, 443-803 Gyeonggi-do (KR); Park, Sangchul, 443-803 Gyeonggi-do (KR); Cho, Sungchun, 443-803 Gyeonggi-do (KR); Jo, Hongseok, 443-803 Gyeonggi-do (KR)
(74) Representative: Grünecker Patent- und Rechtsanwälte PartG mbB

(56) References cited:
- EP-A1- 1 655 055
- EP-A1- 2 425 829
- EP-A1- 2 789 334
- US-A1- 2006 286 108
- BERNARD P ET AL: "Dapsone and rheumatoid vasculitis leg ulcerations", JOURNAL OF THE AMERICAN ACADEMY OF DERMATOLOGY, MOSBY, INC, US, vol. 18, no. 1, 1 January 1988 (1988-01-01), pages 140-141, XP023131975, ISSN: 0190-9622, DOI: 10.1016/S0190-9622(88)80049-5 [retrieved on 1988-01-01]
- "Alopecia areata", British Medical Journal , 24 February 1979 (1979-02-24), XP55248826, Retrieved from the Internet: URL:http://www.ncbi.nlm.nih.gov/pmc/articl es/PMC1598014/pdf/brmedj00061-0005.pdf [retrieved on 2016-02-09]
- RUSTIN M H A ET AL: "FOLLICULAR MUCINOSIS PRESENTING AS ACUTE DERMATITIS AND RESPONSE TO DAPSONE", CLINICAL AND EXPERIMENTAL DERMATOLOGY, vol. 14, no. 5, 1989, pages 382-384, XP002754064, ISSN: 0307-6938
- MOCHIDA O ET AL: "Preventive effect of dapsone on renal scarring following mannose-sensitive piliated bacterial infection", CHEMOTHERAPY, S. KARGER AG, CH, vol. 44, no. 1, 1 January 1998 (1998-01-01), pages 36-41, XP002903496, ISSN: 0009-3157, DOI: 10.1159/000007088
- LEONARD J N ET AL: "DAPSONE AND THE RETINA", LANCET, vol. 1, no. 8269, 1982, page 453, XP002754065,

## Description

### BACKGROUND

### 1. Field

The present disclosure relates to a composition for stimulating neovascularization at a site in a subject, wherein the composition includes dapsone, and methods of use thereof.

### 2. Description of the Related Art

Dapsone (diaminodiphenylsulfone or, also, referred to as "DDS"), of which the IUPAC name is 4-[(4-aminobenzene)sulfonyl]aniline, is an antibacterial most commonly used in combination with rifampicin and clofazimine in multidrug therapy (MDT) for the treatment of *Mycobacterium leprae* infections (leprosy). It is also a second-line treatment for prevention or prophylaxis against Pneumocystis pneumonia (PCP) caused by *Pneumocystis jirovecii* in an HIV patient whose CD4 counts are below 200/mm³. Dapsone is also used in combination with pyrimethamine in the treatment of malaria. Dapsone is commercially available in both topical and oral formulations.

Despite such known effects of dapsone, an effect of dapsone on stimulating neovascularization in a tissue has not been disclosed.

### SUMMARY

Provided is a composition for stimulating vascularization in a subject, the composition including dapsone or N-acetyl-dapsone, its pharmaceutically acceptable salt, a solvate, a polymorph, or a combination thereof.

Provided is a composition for prevention or treatment of symptoms related to a decrease in vascularization in a subject, the composition including dapsone or N-acetyl-dapsone, its pharmaceutically acceptable salt, a solvate, a polymorph, or a combination thereof.

Provided is dapsone or N-acetyl-dapsone for use in a method of preventing or treating symptoms related to a decrease in neovascularization in a subject, the method including administering dapsone or N-acetyl-dapsone, its pharmaceutically acceptable salt, a solvate, a polymorph, or a combination thereof to a site of the subject exhibiting decreased neovascularization.

Also provided is dapsone or N-acetyl-dapsone for use in a method of stimulating neovascularization at a site in a subject, the method including administering dapsone or N-acetyl-dapsone, its pharmaceutically acceptable salt, a solvate, a polymorph, or a combination thereof to the subject.

Additional aspects, including related methods and compositions, will be set forth in part in the description which follows and, in part, will be apparent from the description, or may be learned by practice of the presented exemplary embodiments.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 is a set of micrographs showing the results of immunohistochemical assay performed on a trachea whole-mount of mouse;
FIG. 2 is a graph showing Alexa 594 fluorescence (wavelength = 574 nm) intensity (pixel) of sites corresponding to PECAM-1 in FIG. 1;
FIG. 3 is a set of micrographs showing the results of immunohistochemical assays performed on a mouse ear skin whole-mount;
FIG. 4 is a graph showing an intensity (pixel) of Alexa594 fluorescence (wavelength = 574 nm) of sites corresponding to PECAM-1 in FIG. 3;
FIG. 5 is a set of micrographs showing the results of fluorescein-lectin tracking and immunohistochemical assays performed on a kidney section of a mouse in a control group;
FIG. 6 is a set of micrographs showing the results of fluorescein-lectin tracking and immunohistochemical assays performed on a kidney section of a mouse in an experimental group;
FIG. 7 is a set of micrographs showing the results of fluorescein-lectin tracking and immunohistochemical assays performed on a pancreas section of a mouse in a control group;
FIG. 8 is a set of micrographs showing the results of fluorescein-lectin tracking and immunohistochemical assays performed on a pancreas section of a mouse in an experimental group;
FIG. 9 is a graph showing the analysis results of gene expression in soleus muscles of a mouse in a control group and a mouse in an experimental group by using a microarray; and
FIG. 10 is a set of graphs showing the analysis results of protein expressed in the soleus muscles of a mouse in the control group and a mouse in the experimental group by using a phosphorylation-specific antibody array in which an antibody specific to phosphorylated protein is fixed.

### DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms. Accordingly, the exemplary embodiments are merely described below, by referring to the figures, to explain aspects. As used herein, the term "and/or" includes any and all combinations of one or more of the associated listed items. Expressions such as "at least one of", when preceding a list of elements, modify the entire list of elements and do not modify the individual elements of the list.

Provided is dapsone or N-acetyl-dapsone for use in a method of stimulating neovascularization at a site in a subject, or preventing or treating symptoms related to a decrease in neovascularization in a subject, the method including administering dapsone or N-acetyl-dapsone, its pharmaceutically acceptable salt, a solvate, a polymorph, or a combination thereof to the subject. Also provided are compositions useful in such a method. Dapsone is also referred to as diaminodiphenylsulfone or DDS, and its IUPAC name is 4-[(4-aminobenzene)sulfonyl] aniline. Dapsone may have a structure represented by Formula 1.

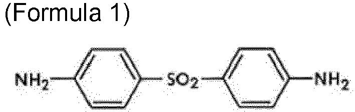

Dapsone may be commercially available, synthesized in-house, or extracted from a natural product. In order to improve its water-insoluble characteristics, dapsone may be mixed with water and then sterilized at high pressure. The high-pressure sterilization treatment may be performed by using any method known in the art. The high-pressure sterilization may be performed, for example, at a temperature of 121°C and a pressure of 151 psi for about 15 minutes to about 20 minutes. Despite having water-insoluble characteristics, dapsone may be dissolved in water to achieve a specific concentration by mixing dapsone with water and sterilizing the same at high pressure. Also, an effective concentration of dapsone in blood may be within an appropriate range when administering to a mouse. In addition, by the high-pressure sterilization treatment, activity of dapsone, that is, antibiotic effect against bacteria, may be preserved. N-acetyl-dapsone may be N-monoacetyl-dapsone or N,N'-diacetyl-dapsone. N-acetyl-dapsone is a metabolite of dapsone in a cell which is commercially available but also may be synthesized in-house. The synthesis may be performed by, for example, N-acetylation of dapsone.

The term "pharmaceutically acceptable salt" used herein may refer to a salt that does not generate a serious adverse effect in an organism to which a compound is administered, and that does not damage (substantially reduce) the biological activity and/or physical properties of the compound. The salt may be, for example, an inorganic acid salt, an organic acid salt, or a metal salt. Examples of the inorganic acid salt may include hydrochlate, hydrobromate, phosphate, sulfate, or disulfate. Examples of the organic acid salt may include formate, nitrate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, methanesulfonate, ethanesulfonate, benzenesulfonate, p-toluenesulfonate, or aspartate. Examples of the metal salt may include a calcium salt, a sodium salt, a magnesium salt, a strontium salt, or a potassium salt. "Solvate" means a physical association of a compound of this disclosure with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Examples of suitable solvates include hydrates, ethanolates, methanolates, and the like. As used herein, "polymorph" refers to a crystalline compound having more than one crystal structure, e.g., resulting from differences in molecular packing and/or molecular conformation of the compound in the solid state. When polymorphism exists as a result of difference in crystal packing it is called packing polymorphism. Polymorphism can also result from the existence of different conformers of the same molecule in conformational polymorphism. In pseudopolymorphism the different crystal types are the result of hydration or solvation. One exemplary way of characterizing a polymorph is via its unique X-ray powder diffraction (XRPD) pattern.

The dapsone or pharmaceutically acceptable salt, solvate, polymorph, or combination thereof is an active component for stimulating neovascularization at a site in a subject, which may be included in a composition (e.g., pharmaceutical composition) in any therapeutically effective amount (e.g., an amount or concentration sufficient to stimulate neovascularization). The term "therapeutically effective amount" denotes an amount of a dapsone or pharmaceutically acceptable salt, solvate, polymorph, or combination thereof that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgment of the attending medical or veterinary practitioner, and other factors such a type of a cell. The therapeutically effective amount may be appropriately selected by one of ordinary skill in the art according to a cell or a subject. The therapeutically effective amount may be in a range of about 1 µg to about 1,000 mg, for example, about 0.1 mg to about 1,000 mg, about 0.1 mg to about 500 mg, about 0.1 mg to about 100 mg, about 0.1 mg to about 50 mg, about 0.1 mg to about 25 mg, about 1 mg to about 1,000 mg, about 1 mg to about 500 mg, about 1 mg to about 100 mg, about 1 mg to about 50 mg, about 1 mg to about 25 mg, about 5mg to about 1,000 mg, about 5 mg to about 500 mg, about 5 mg to about 100 mg, about 5 mg to about 50 mg, about 5 mg to about 25 mg, about 25 mg to about 100 mg, about 10mg to about 1,000 mg, about 10 mg to about 500 mg, about 10 mg to about 100 mg, about 10 mg to about 50 mg, or about 10 mg to about 25 mg per the total weight of the composition.

The neovascularization may refer to formation of functional microvascular networks having red blood cell perfusion. The neovascularization includes protrusion and overgrowth of capillary buds, neovascularization characterized by sprouts from pre-existing blood vessels (angiogenesis), and vascularization, which is de novo formation of endothelial cells from mesoderm cell precursors. Due to stimulation of neovascularization, the neovascularization may increase at a site in a subject or a decrease in the neovascularization may be prevented. The neovascularization stimulated by the composition may have at least one of the characteristics including recruitment of perivascular support cells, non-leakiness of vessels, and well-defined arborization.

Regarding the composition, the subject may have symptoms related to a decrease in neovascularization. The phrase "a decrease in neovascularization" may refer to a decrease in the blood vessel density per unit volume of tissue, for example, mm3 tissue, compared to the blood vessel density per unit volume of the same type of tissue of a control person. The control person may be a younger person, for example a person aged between about 20 years old and about 50 years old, for example, about 20 years old and about 45 years old, about 20 years old and about 40 years old, about 20 years old and about 35 years old, about 20 years old and about 30 years old, about 20 years old and about 25 years old, about 30 years old and about 50 years old, about 30 years old and about 45 years old, about 30 years old and about 40 years old, about 25 years old and about 35 years old, or about 25 years old and about 30 years old. The control person may be in normal health. Normal health may include exhibiting no symptoms related to a decrease in neovascularization such as ischemic symptoms. The composition may be prepared for prevention or treatment of symptoms related to a decrease in neovascularization at a site in a subject. The subject may have an age-associated degenerative tissue. The age-associated degenerative tissue may skin, kidney, pancreas, blood vessel, or heart. Skin tends to be easily damaged due to a decrease in skin thickness by aging, and the decrease in epidermal tissue increases a risk of low-body temperature, algidity, or damage with respect to pressure. One of the factors causing skin disorders may be an exposure to sun-light for a long period of time, and the skin disorders may often include an ulcer, keratosis, skin cancer, fungal infection, dermatitis, and skin coloration. Changes in kidneys according to the aging are relatively significant compared to any other tissues or organs in the body. In case of an elderly person, the change in kidney further increases when an external injury or disease occurs while a kidney function declines in general, and thus vulnerability of the elderly person may increase. Particularly, function of glomerular capillaries may be lost, and thus permeation of metabolic waste may become difficult, which may result in accumulation of the waste in the body and causes various diseases. Glycometabolism, thyroid hormone secretion, and estrogen secretion may undergo changes associated with aging. In particular, when a function of the pancreas declines, the ability of performing glucose metabolism decreases, sensitivity with respect to insulin decreases, and thus high glucose level in blood may easily be a result. This is because the level of blood sugar increases during fasting time due to pancreatic beta-cell with slowly secreting insulin, and thus, in sufficient amount of the insulin secretion to convert glycogen to glucose. The subject may be, for example, an elderly person aged 60 years or older, 70 years or older, 80 years or older, or 90 years or older. The elderly person may be in normal health. Normal health may include exhibiting no ischemic symptoms.

The symptoms related to decreased neovascularization, or symptoms that are treatable by increasing neovascularization, may include ischemia, or a disease or a state related to (e.g., resulting from) ischemia. The ischemia limits blood supply to a tissue and thus results in a deficit of oxygen and glucose that are necessary in cell metabolism. The ischemia is generally caused by problems in blood vessels and causes damage or dysfunction to tissues accordingly. In some cases, the ischemia may also denote local anemia in a given part of a body caused by congestion such as vasoconstriction, thrombosis, or embolism. The ischemia may include cardiac ischemia, bowel ischemia, brain ischemia, limb ischemia, eye ischemia, or skin ischemia. The ischemia may be caused by occlusion or trauma. The occlusion may include, for example, thromboembolism caused by blood clots and exogenous substance in blood circulation, or, for example, embolism such as amniotic fluid embolism. Extreme traumatic injury may cause partial or the whole occlusion of vessels. The skin ischemia may include scleroderma or wound. The skin ischemia may include decubitus ulcers, pressure ulcers, surgical incisions, traumatic tissue injuries, diabetic ulcers, burns, skin grafts, or combinations thereof. The eye ischemia may be retinal vascular occlusive disorders including central retinal vein occlusion and branch retinal vein occlusion. Also, the symptoms may include chronic kidney disease, cerebral infarction, myocardiac infarction (MI), angina pectoris, coronary heart disease, diabetic complications, ischemic limb disease, cerebral ischemia, conditions of vascular inflammation, arteriosclerosis, avascular necrosis, hair loss, degenerative tissue, and erectile dysfunction. The chronic kidney disease may include nondiabetic chronic kidney disease.

The composition comprising dapsone or N-acetyl-dapsone, its pharmaceutically acceptable salt, a solvate, a polymorph, or a combination thereof may further include a second angiogenic agent. The second angiogenic agent may be selected from the group consisting of PDGF, G-CSF, recombinant human erythropoietin, bFGF, VEGF, angiopoietin, and placental growth factor (PIGF).

The composition may be a pharmaceutical formulation to be administered topically at a site in a subject. Examples of the site in a subject may include skin, eye, lung, blood vessel, kidney, heart, scalp, degenerative tissue, or male genitalia.

The composition may be a pharmaceutical or functional food composition. The pharmaceutical composition may include a pharmaceutically acceptable diluent or carrier. The carrier may be a diluting agent, a disintegrating agent, a binding agent, a lubricating agent, or a combination thereof. The diluting agent may be microcrystalline cellulose, lactose, low-substituted hydroxypropyl cellulose, hydroxypropyl cellulose, or a combination thereof. The disintegrating agent may be sodium starch glycolate, anhydrous dibasic calcium phosphate, or a combination thereof. The binding agent may be polyvinylpyrrolidone, low-substituted hydroxypropyl cellulose, hydroxypropyl cellulose, or a combination thereof. The lubricating agent may be magnesium stearate, silicon dioxide, talc, or a combination thereof. The functional food can be, e.g., solid, liquid, semisolid, or a combination thereof. The term "functional food" also may encompass edible and drinkable nutritional supplements.

The composition may have a formulation for an oral or parenteral administration. The formulation for an oral administration may be granules, powders, solutions, tablets, capsules, dry syrup, or a combination thereof. The formulation for a parenteral administration may be injections or external skin application. The external skin application may be a cream, gel, ointment, skin emulsion, skin suspension, transdermal patch, drug-containing bandage, lotion, or a combination thereof.

The term "prevention" used herein denotes all practices that suppress symptoms related to a decrease in neovascularization, or delay occurrence of a disease or condition associated therewith, by administration of a composition.

The term "treatment" used herein denotes all practices that relieve, to any degree, symptoms related to a decrease in neovascularization.

Without wishing to be bound by any particular theory or mechanism of action, the stimulating of neovascularization by administration of dapsone or N-acetyl-dapsone, its pharmaceutically acceptable salt, a solvate, a polymorph, or a combination thereof may be caused by increased signal transduction of VEGF-A and VEGFR-2 pathways. Thus, the methods and compositions described herein may increase signal transduction of VEGF-A and VEGFR-2 pathways.

The administration of the dapsone or N-acetyl-dapsone, its pharmaceutically acceptable salt, a solvate, a polymorph, or a combination thereof to the subject may be performed by using any method known in the art. The administration may be performed by directly administering the compound to the subject by various routes including intravenous, intramuscular, oral, transdermal, mucosal, intranasal, intratracheal, epidermal application or introduction, or subcutaneous administration. The administration may be performed systematically or topically. The administration may be performed topically at a site having the symptoms related to a decrease in neovascularization. Examples of the site in a subject may include skin, eye, lung, blood vessel, kidney, heart, scalp, degenerative tissue, or male genitalia.

The subject may have symptoms related to a decrease in neovascularization. The method may be effected by stimulation of neovascularization caused by the dapsone or N-acetyl-dapsone, its pharmaceutically acceptable salt, a solvate, a polymorph, or a combination thereof. The stimulation of neovascularization may be caused by increasing signal transduction of VEGF-A and VEGFR-2 pathways. The method may increase signal transduction of VEGF-A and VEGFR-2 pathways.

The subject may be a mammal, for example, a human, a cow, a horse, a pig, a dog, a sheep, a goat, a mouse, a rat, or a cat.

The method may further include administering a second angiogenic agent. The second angiogenic agent may be selected from the group consisting of PDGF, G-CSF, recombinant human erythropoietin, bFGF, VEGF, angiopoietin, and placental growth factor (PIGF). The administering of the second angiogenic agent may be performed before, at the same time, or after the administering of the dapsone or N-acetyl-dapsone, its pharmaceutically acceptable salt, a solvate, a polymorph, or a combination thereof.

A dose of the dapsone or N-acetyl-dapsone, its pharmaceutically acceptable salt, a solvate, a polymorph, or a combination thereof for the administration may be in a range of about 0.1 mg to about 1,000 mg, for example, about 0.1 mg to about 500 mg, about 0.1 mg to about 100 mg, about 0.1 mg to about 50 mg, about 0.1 mg to about 25 mg, about 1 mg to about 1,000 mg, about 1 mg to about 500 mg, about 1 mg to about 100 mg, about 1 mg to about 50 mg, about 1 mg to about 25 mg, about 5 mg to about 1,000 mg, about 5 mg to about 500 mg, about 5 mg to about 100 mg, about 5 mg to about 50 mg, about 5 mg to about 25 mg, about 10mg to about 1,000 mg, about 10 mg to about 500 mg, about 10 mg to about 100 mg, about 10 mg to about 50 mg, or about 10 mg to about 25 mg per kg of the weight of the subject per day. A period of the administration may be about 2 months or more, 3 months or more, 6 months or more, or 1 year or more. The administration may be performed every day for about 2 months to about 1 year, for about 2 months to about 10 months, for about 2 months to about 6 months, or for about 2 months to about 4 months.

One or more embodiments will be described in further detail with reference to the following examples. These examples are for illustrative purposes only.

### Example:

C57BL/6 mice (hereinafter, also referred to as "B6") at ages of 7 months and 24 months (female n=18, male n=12, Korean Basic Science Institute) were maintained according to the guideline of the Laboratory Animal Research Center of Sungkyunkwan University School of Medicine. The mice were allowed to have free access to water and feed. Each group of mice (n=5) drank 0.0, 0.5, or 2.0 mg/kg/day of dapsone (available from Tai Guk Pharm. Co.) for 3 months.

After 3 months, ear skin from both ears, trachea, both retinas, both kidneys, and pancreas were obtained from a mouse selected from each group of mice. In order to obtain these organs, the mouse was anesthetized by intraperitoneal injection of 100 mg/kg of phenobarbital sodium (Entobal®) based on the weight of the mouse, 200 ml of 1% paraformaldehyde was flowed through the heart to remove the blood in the body of the mouse. The organs were surgically obtained, and tissue from each of the organs was fixed in 4% of paraformaldehyde for 24 hours to prepare a frozen section having a thickness of 10 µm. Immunofluorescence staining using anti-PECAM-1 antibody was performed on a sample of the frozen section. For the immunofluorescence staining, the sample was blocked in a solution containing 5% donkey serum in phosphate buffered solution (PBS) containing 0.3% Triton-X 100 (0.3% PBST) for 1 hour. The anti-PECAM-1 antibody produced from American hamster was added to the solution to allow a primary reaction with antigen of the tissue at 4°C for 12 hours. The sample was washed 6 times for 10 minutes with 0.3% PBS-T, and an anti-American hamster-Alexa594 antibody was added to 0.3 PBS-T to allow a secondary reaction with the anti-PECAM-1 antibody at 4°C for 12 hours. The sample was washed 6 times for 10 minutes, attached to a slide glass, and thus image of the stained sample was obtained by a Carl Zeiss LSM710 confocal microscope. Each of the images was sequentially taken with respect to the tissue having a thickness in a range of about 100 µm to about 300 µm as an axis, and then all the sub-images were projected in a z-axis direction. Thus the image is a projection image. Platelet endothelial cell adhesion molecule (PECAM-1) is also referred to as cluster of differentiation 31 (CD31), which is found in platelets or monocytes and on a surface of some types of T-cells, and constitutes most of endothelial cell intercellular junction. PECAM-1 is mainly used to prove existence of endothelial cells in histological tissue sections in immunohistochemical assay. The measured fluorescent density denotes a density of endothelial cells, that is, blood vessels. Also, in terms of kidney tissue sections and pancreas tissue section, Fluorescein labeled *Lycopersicon esculentum* (tomato) lectin (hereinafter, also referred to as "Fluorescein-lectin") was used as a tracer to verify blood vessel functionality of the sample. Lectin is a marker of blood vessels and microglial cells which enables verification of blood vessels having functionality of blood flow. 10 mg/kg of Fluorescein-lectin was injected to orbital venous sinus of each of the mice before obtaining the tissue. Fifteen minutes after the Fluorescein-lectin injection, 200 ml of 1% paraformaldehyde was flowed through the heart to remove blood in the body of the mouse. Each of the tissues thus obtained was fixed in 4% of paraformaldehyde for 24 hours, and a frozen section having a thickness of 10 µm was prepared using the fixed tissue. Fluorescent staining of PECAM was simultaneously performed to distinguish blood vessels where actual blood flowed and blood vessels where no blood flowed. Imaging of the stained sample was obtained under a Carl Zeiss LSM710 confocal microscope.

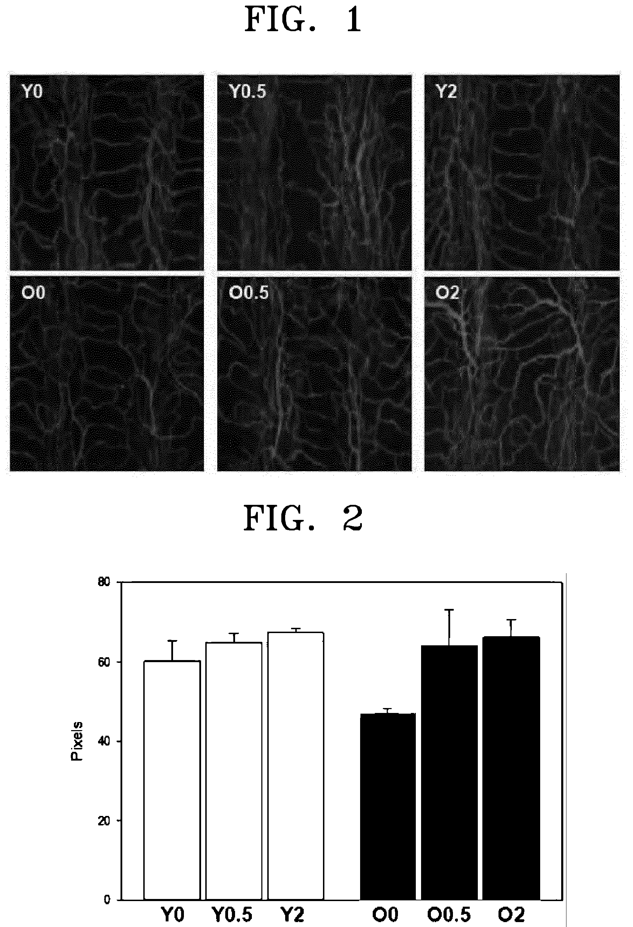

FIG. 1 shows the results of immunohistochemical assay performed on a trachea whole-mount of mouse. In FIG. 1, Y0, Y0.5, and Y2 from upper left to right refer to groups of 7 months-old C57BL/6 mice that respectively drank 0.0 mg/kg/d, 0.5 mg/kg/d, and 2.0 mg/kg/d of dapsone for 3 months (10 months-old at the time of sacrifice), and O0, 00.5, and 02 from lower left to right refer to groups of 27 months-old C57BL/6 mice that respectively drank 0.0 mg/kg/d, 0.5 mg/kg/d, and 2.0 mg/kg/d of dapsone for 3 months (30 months-old at the time of sacrifice).
FIG. 2 is a graph showing Alexa 594 fluorescence (wavelength = 574 nm) intensity (pixel) of sites corresponding to PECAM-1 in FIG. 1. The intensity of fluorescence is related to a density of blood vessels. The fluorescence intensity (pixel number) measurement was performed by using Image J program (available online << image.nih.gov>>; authored by Wayne Rasband of the Research Services Branch, National Institutes of Health, Bethesda, MD). ImageJ is a public domain Java image processing and analysis program inspired by NIH Image for the Macintosh. It can read many image formats including TIFF, GIF, JPEG, BMP, DICOM, FITS and 'raw'. It supports 'stacks' (and hyperstacks), a series of images that share a single window. It is multithreaded, so time-consuming operations such as image file reading can be performed in parallel with other operations.

As shown in FIGS. 1 and 2, the control group of old mice that did not drink dapsone (O0) had a low blood vessel density compared to that of the control group of young mice that did not drink dapsone (Y0). Also, within the groups of young mice, no significant difference in blood vessel density was noted between those of the control group (Y0) and the experimental groups (Y0.5 and Y2). However, within the groups of old mice, a significant difference in blood vessel density was noted between those of the control group (O0) and the experimental groups (00.5 and 02). Particularly, when the old mice drank 0.5 mg/kg/d or 2.0 mg/kg/d of dapsone for 3 months, the blood vessel density thereof was similar to or higher than that of the control group of young mice (Y0) that did not drink dapsone, and thus it was confirmed that dapsone treatment increased blood vessel density of the old mice to a level before aging.

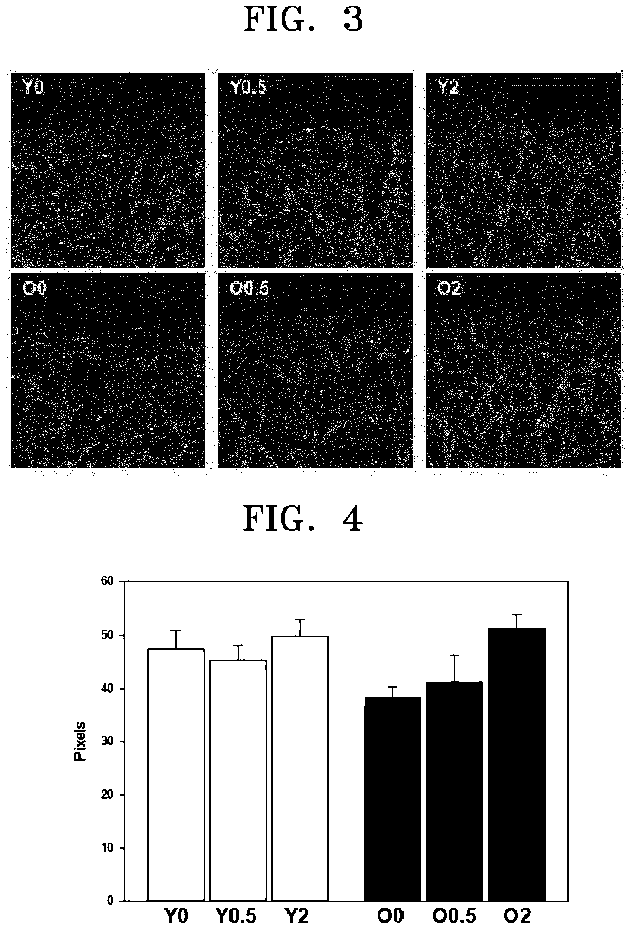

FIG. 3 shows the results of an immunohistochemical assay performed on a mouse ear skin whole-mount. In FIG. 3, Y0, Y0.5, and Y2 from upper left to right refer to groups of 7 months-old C57BL/6 mice (young mice) that respectively drank 0.0 mg/kg/d, 0.5 mg/kg/d, and 2.0 mg/kg/d of dapsone for 3 months (10 months-old at the time of sacrifice), and O0, 00.5, and 02 from lower left to right refer to groups of 27 months-old C57BL/6 mice (old mice) that respectively drank 0.0 mg/kg/d, 0.5 mg/kg/d, and 2.0 mg/kg/d of dapsone for 3 months (30 months-old at the time of sacrifice).

FIG. 4 is a graph showing an intensity (pixel) of Alexa594 fluorescence (wavelength = 574 nm) which is a site corresponding to PECAM-1 in FIG. 3. The intensity of fluorescence is related to a density of blood vessels. The fluorescence intensity (pixel number) measurement was performed by using Image J program (available online << image.nih.gov>>; authored by Wayne Rasband of the Research Services Branch, National Institutes of Health, Bethesda, MD). As shown in FIGS. 3 and 4, the control group of old mice that did not drink dapsone (O0) had a low blood vessel density compared to that of the control group of young mice that did not drink dapsone (Y0). Also, within the groups of young mice, no significant difference in blood vessel density was noted between those of the control group (Y0) and the experimental groups (Y0.5 and Y2). However, within the groups of old mice, a significant difference in blood vessel density was noted between those of the control group (O0) and the experimental group(O2), not the O0.5 group. Particularly, when the old mice drank 2.0 mg/kg/d of dapsone for 3 months, the blood vessel density thereof was similar to or higher than that of the control group of young mice (Y0) that did not drink dapsone, and thus it was confirmed that a blood vessel density of the old mice was increased to a level before aging.

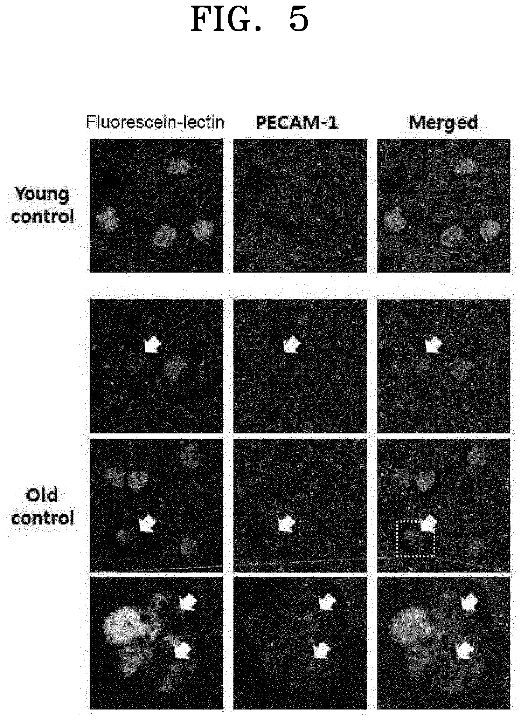

FIG. 5 shows the results of fluorescein-lectin tracking and immunohistochemical assay performed on a kidney section of a mouse in a control group. In FIG. 5, the first upper row is a young control group, and the second to fourth rows are old control groups. In FIG. 5, the second and third rows are of a plurality of samples, and the fourth row is an enlarged view of the dot-lined box in the third row. In FIG. 5, arrows indicate blood vessels having decreased functionality. That is, in FIG. 5, blood vessels that are immunofluorescent stained with red fluorescence structurally existed but were negative in Fluorescein-lectin, and thus it was confirmed that functionality of the blood vessels had decreased. Also, an enlarged view of the dot-lined box in the third row is shown in the fourth row.

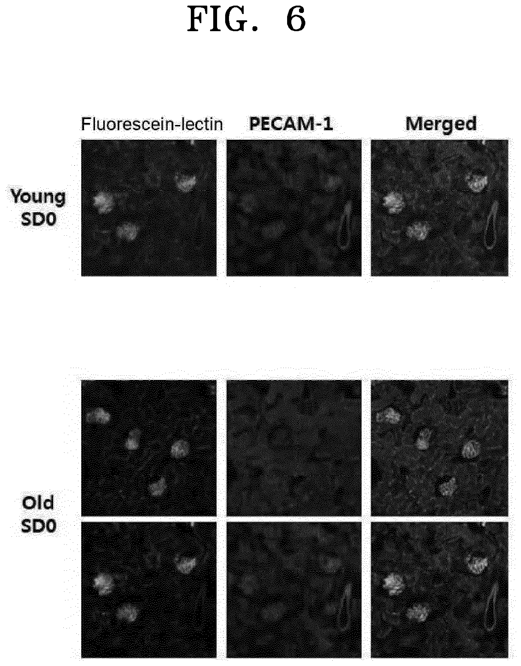

FIG. 6 shows the results of fluorescein-lectin tracking and immunohistochemical assay performed on a kidney section of mice from experimental groups. In FIG. 6, each group of mice had 2.0 mg/kg/d of dapsone for 3 months. In FIG. 6, the first to third rows are arranged in the same manner as the first to third rows in FIG. 5: the first upper row is a young experimental group, and the second and third rows are old experimental groups. In FIG. 6, the second and third rows are of a plurality of samples.

As shown in FIG. 5, the kidney tissue of the old mice in the control group had decreased functionality compared to that of the kidney tissue of the young mice. In FIG. 6, the second and third rows demonstrate that the blood vessel functionality of the old mice was recovered to the similar level of the control group by allowing the old mice to drink 2.0 mg/kg/d of dapsone.

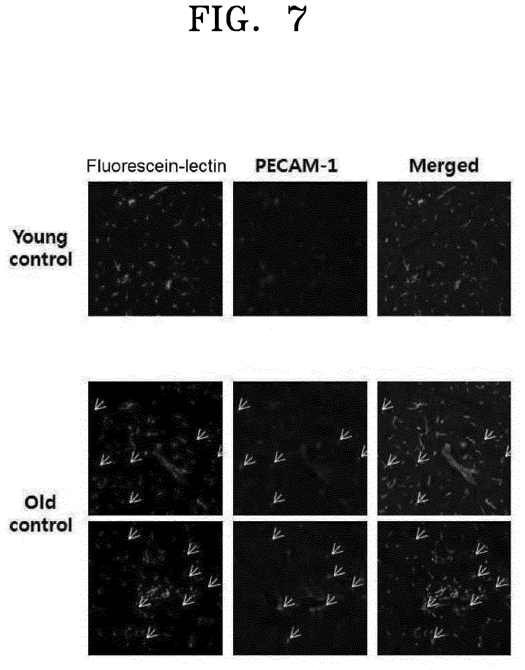

FIG. 7 shows the results of fluorescein-lectin tracking and immunohistochemical assay performed on a pancreas section of a mouse in a control group. In FIG. 7, the first upper row is a young control group, and the second and third rows are a plurality of old control groups.

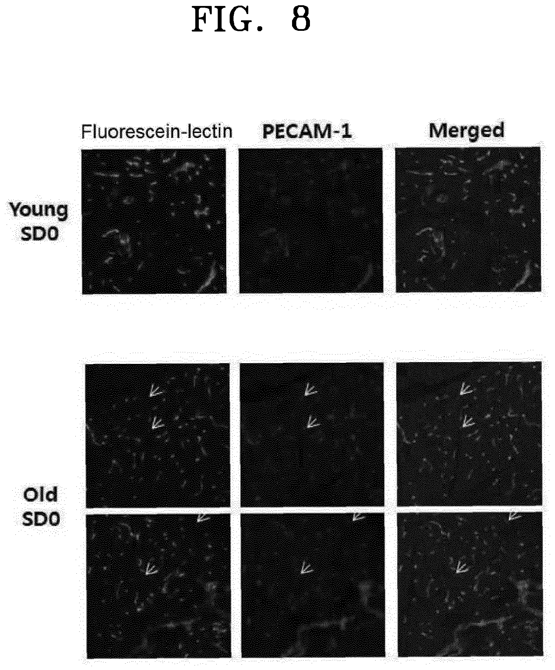

FIG. 8 shows the results of fluorescein-lectin tracking and immunohistochemical assay performed on a pancreas section of a mouse in an experimental group. In FIG. 8, each group of mice had 2.0 mg/kg/d of dapsone for 3 months. In FIGS. 7 and 8, arrows indicate blood vessels having decreased functionality. Blood vessels that are immunofluorescent stained with red fluorescence structurally existed but were negative in Fluorescein-lectin, and thus it was confirmed that blood did not flow through the blood vessels which had decreased functionality. Thus, in FIGS. 7 and 8, blood vessels having decreased functionality are green fluorescent negative, as indicated by the arrows. Comparison of Figs. 7 and 8 shows that the amount of blood vessels with decreased functionality in the old mice was reduced by treatment with dapsone. This can be supported by measuring the density of perfused blood vessels in the pancreas sections according to FIGS.7 and 8.

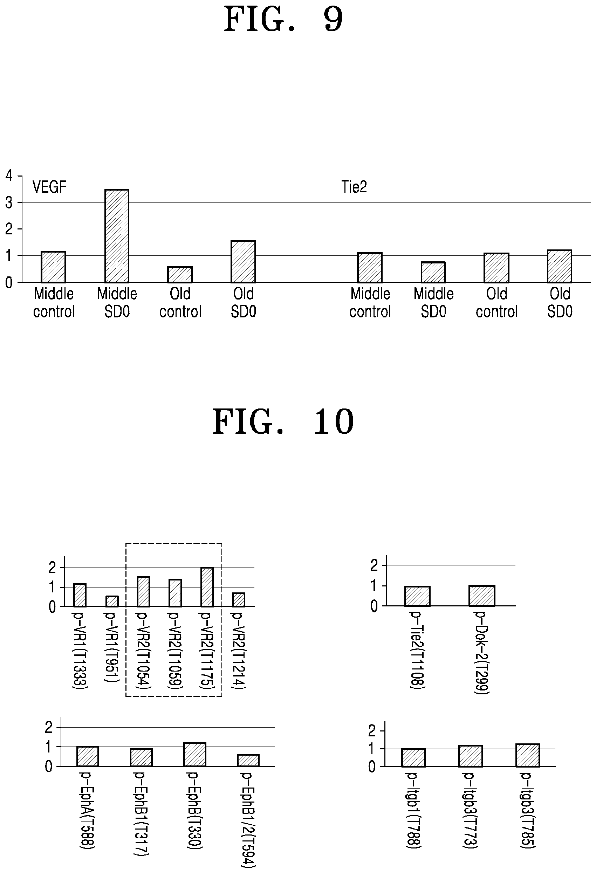

FIG. 9 shows the analysis results of gene expression in soleus muscles of a mouse in a control group and a mouse in an experimental group by using a microarray. In FIG. 9, a vertical axis denotes an expression amount of VEGF or Tie2 gene (fluorescent intensity: arbitrary unit), and a horizontal axis denotes a mouse subject. In FIG. 9, "middle" indicates 15 month-old mice; "old" indicates 20 month-old mice; "SD0" indicates a group of mice that had 2.0mg/kg/d of dapsone for 3 months, and "control" indicates a group of mice that did not have dapsone. The microarray analysis was performed by using Agilent's Mouse Oligo Microarray (44K, Agilent Technology). 5 months-old, 15 months-old, and 20 months-old C57BL/6J mice had 2mg/kg/d of dapsone for 3 months. Since dapsone does not dissolve well in water, dapsone was mixed with water and high-pressure sterilized to be dissolved in water. After the 3 months, the 8 months-old, 18 months-old, and 23 months-old mice were anesthetized by injecting Entobar (sodium pentobarbital, 100 mg/kg per body weight), and then soleus muscle taken therefrom for microarray analysis was frozen. Ribonucleic acid was isolated from the muscle and used for the microarray analysis.

As shown in FIG. 9, expression of VEGF increased in the experimental groups compared to that of the control groups. This indicates that the dapsone may increase neovascularization through the VEGF and VEGR pathway, but not angiopoietin and its receptor Tie2, since the level of VEGF significantly changed, while the level of Tie2 did not change significantly.

FIG. 10 shows the analysis results of protein expressed in the soleus muscles of a mouse in the control group and a mouse in the experimental group by using a phosphorylation-specific antibody array in which an antibody specific to phosphorylated protein is fixed. In FIG. 10, a vertical axis denotes the amount of protein expressed in the experimental group compared to the control group (fluorescent intensity: arbitrary unit), and a horizontal axis denotes the type of phosphorylated protein measured, where the description in parentheses informs residue and location of the phosphorylated amino acid. In FIG. 10, the experimental group 20 month-old C57BL/6J mice had 2.0mg/kg/d of dapsone for 3 months, and the control group did not receive dapsone but was otherwise maintained in the same manner as the experimental group. The microarray analysis was performed by using a Phospho Explorer Antibody array (1312 antibodies, FULL MOON Biosystems), soleus muscle of the mice was obtained, protein was isolated therefrom, and used for the microarray analysis. In FIG. 10, p-VR1 and p-VR2 respectively denote phosphorylated VEGFR1 and VEGFR2.

As shown in FIG. 10, T1175, T1059, and T1054 of VEGFR2 had significantly increased phosphorylation compared to that of the control group, and thus it is deemed as VEGFR2 was activated. These results show that the neovascularization signaling by inducing phosphorylated VEGFR2 signal was transmitted through the activated VEGFR2.

As described above, a composition for stimulating neovascularization at a site in a subject may be used to stimulate neovascularization at a site in a subject. Also, a composition for preventing or treating symptoms related to decrease in neovascularization at a site may be used to prevent or treat symptoms related to decrease in neovascularization. Yet also a method of preventing or treating symptoms related to decrease in neovascularization at a site in a subject may be used to efficiently prevent or treat symptoms related to decrease in neovascularization at a site in a subject. Still also, a method of stimulating neovascularization at a site in a subject may be used to efficiently stimulate neovascularization at a site in a subject.

It should be understood that the exemplary embodiments described herein should be considered in a descriptive sense only. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope as defined by the following claims.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A composition for use in stimulating vascularization in a subject, the composition comprising dapsone or N-acetyl-dapsone; a pharmaceutically acceptable salt thereof; a solvate; a polymorph; or a combination thereof.

2. The composition for the use of claim 1, wherein the subject has symptoms related to a decrease in vascularization.

3. The composition for the use of claim 2, wherein the symptoms related to a decrease in vascularization are ischemia or a disease that causes ischemia.

4. The composition for the use of claim 3, wherein the ischemia is skin ischemia or eye ischemia.

5. The composition for the use of claim 4, wherein the skin ischemia is scleroderma or a wound, the wound comprises decubitus ulcers, pressure ulcers, surgical incisions, traumatic tissue injuries, diabetic ulcers, burns, skin grafts, or a combination thereof, and the eye ischemia is a retinal vascular occlusive disorder.

6. The composition for the use of claim 2, wherein the symptoms comprise nondiabetic chronic kidney diseases, cerebral infarction, myocardiac infarction (MI), angina pectoris, coronary heart disease, diabetic complications, ischemic limb diseases, cerebral ischemia, conditions of vascular inflammation, arteriosclerosis, avascular necrosis, hair loss, or erectile dysfunction.

7. A composition for use in preventing or treating symptoms related to a decrease in vascularization in a subject, the composition comprising dapsone or N-acetyl-dapsone; a pharmaceutically acceptable salt thereof; a solvate; a polymorph; or a combination thereof.

8. The composition for the use of claim 7, wherein the symptoms related to a decrease in vascularization are ischemia or a disease that causes ischemia.

9. The composition for the use of claim 8, wherein the ischemia is skin ischemia or eye ischemia.

10. The composition for the use of claim 9, wherein the skin ischemia is scleroderma or a wound, the wound comprises decubitus ulcers, pressure ulcers, surgical incisions, traumatic tissue injuries, diabetic ulcers, burns, skin grafts, or a combination thereof, and the eye ischemia is a retinal vascular occlusive disorder.

11. The composition for the use of claim 7, wherein the symptoms comprise nondiabetic chronic kidney diseases, cerebral infarction, myocardiac infarction (MI), angina pectoris, coronary heart disease, diabetic complications, ischemic limb diseases, cerebral ischemia, conditions of vascular inflammation, arteriosclerosis, avascular necrosis, hair loss, or erectile dysfunction.

12. The composition for the use of any one of claims 1 to 11, wherein the dose of the dapsone or N-acetyl-dapsone; the pharmaceutically acceptable salt thereof; the solvate; the polymorph; or the combination thereof is in a range of 0.1 mg to 1000 mg per kg of the weight of the subject.

13. The composition for the use of any one of claims 1 to 12, wherein the composition is a pharmaceutical or a functional food.

14. Dapsone or N-acetyl-dapsone; a pharmaceutically acceptable salt thereof; a solvate thereof; a polymorph thereof; or a combination thereof for use in preventing or treating symptoms related to a decrease in vascularization in a subject.

15. Dapsone or N-acetyl-dapsone; a pharmaceutically acceptable salt thereof; a solvate thereof; a polymorph thereof; or a combination thereof for use in stimulating vascularization at a site in a subject exhibiting decreased vascularization.

## Patentansprüche

1. Zusammensetzung zur Verwendung beim Stimulieren der Vaskularisation bei einem Probanden, wobei die Zusammensetzung Dapson oder N-Acetyldapson; ein pharmazeutisch verträgliches Salz davon; ein Solvat; ein Polymorph oder eine Kombination davon umfasst.

2. Zusammensetzung zur Verwendung nach Anspruch 1, wobei der Proband Symptome aufweist, die mit einer Abnahme der Vaskularisation zusammenhängen.

3. Zusammensetzung zur Verwendung nach Anspruch 2, wobei es sich bei den mit einer Abnahme der Vaskularisation zusammenhängenden Symptomen um Ischämie oder eine Erkrankung, die Ischämie verursacht, handelt.

4. Zusammensetzung zur Verwendung nach Anspruch 3, wobei es sich bei der Ischämie um Hautischämie oder Augenischämie handelt.

5. Zusammensetzung zur Verwendung nach Anspruch 4, wobei es sich bei der Hautischämie um Sklerodermie oder eine Wunde handelt, wobei die Wunde Dekubitalulcera, Druckgeschwüre, chirurgische Einschnitte, traumatische Gewebeverletzungen, diabetische Ulcera, Verbrennungen, Hauttransplantationen oder eine Kombination davon umfasst, und wobei es sich bei der Augenischämie um eine retinale Gefäßverschlusserkrankung handelt.

6. Zusammensetzung zur Verwendung nach Anspruch 2, wobei die Symptome nichtdiabetische chronische Nierenerkrankungen, Hirninfarkt, Myokardinfarkt (MI), Angina pectoris, koronare Herzerkrankung, diabetische Komplikationen, ischämische Erkrankungen der Extremitäten, Hirnischämie, Zustände von Gefäßentzündung, Arteriosklerose, avaskuläre Nekrose, Haarverlust oder erektile Dysfunktion umfassen.

7. Zusammensetzung zur Verwendung beim Vorbeugen oder Behandeln von Symptomen, die mit einer Abnahme der Vaskularisation bei einem Probanden zusammenhängen, wobei die Zusammensetzung Dapson oder N-Acetyldapson; ein pharmazeutisch verträgliches Salz davon; ein Solvat; ein Polymorph oder eine Kombination davon umfasst.

8. Zusammensetzung zur Verwendung nach Anspruch 7, wobei es sich bei den mit einer Abnahme der Vaskularisation zusammenhängenden Symptomen um Ischämie oder eine Erkrankung, die Ischämie verursacht, handelt.

9. Zusammensetzung zur Verwendung nach Anspruch 8, wobei es sich bei der Ischämie um Hautischämie oder Augenischämie handelt

10. Zusammensetzung zur Verwendung nach Anspruch 9, wobei es sich bei der Hautischämie um Sklerodermie oder eine Wunde handelt, wobei die Wunde Dekubitalulcera, Druckgeschwüre, chirurgische Einschnitte, traumatische Gewebeverletzungen, diabetische Ulcera, Verbrennungen, Hauttransplantationen oder eine Kombination davon umfasst, und wobei es sich bei der Augenischämie um eine retinale Gefäßverschlusserkrankung handelt.

11. Zusammensetzung zur Verwendung nach Anspruch 7, wobei die Symptome nichtdiabetische chronische Nierenerkrankungen, Hirninfarkt, Myokardinfarkt (MI), Angina pectoris, koronare Herzerkrankung, diabetische Komplikationen, ischämische Erkrankungen der Extremitäten, Hirnischämie, Zustände von Gefäßentzündung, Arteriosklerose, avaskuläre Nekrose, Haarverlust oder erektile Dysfunktion umfassen.

12. Zusammensetzung zur Verwendung nach einem der Ansprüche 1 bis 11, wobei die Dosis des Dapsons oder N-Acetyldapsons; des pharmazeutisch verträglichen Salzes davon; des Solvats; des Polymorphs oder der Kombination davon in einem Bereich von 0,1 mg bis 1000 mg pro kg Gewicht des Probanden liegt.

13. Zusammensetzung zur Verwendung nach einem der Ansprüche 1 bis 12, wobei es sich bei der Zusammensetzung um ein Pharmakon oder um ein funktionelles Nahrungsmittel handelt.

14. Dapson oder N-Acetyldapson; pharmazeutisch verträgliches Salz davon: Solvat davon; Polymorph davon oder Kombination davon zur Verwendung beim Vorbeugen oder Behandeln von Symptomen, die mit einer Abnahme der Vaskularisation bei einem Probanden zusammenhängen.

15. Dapson oder N-Acetyldapson; pharmazeutisch verträgliches Salz davon; Solvat davon; Polymorph davon oder Kombination davon zur Verwendung beim Stimulieren der Vaskularisation an einer Stelle bei einem Probanden, die verringerte Vaskularisation aufweist.

## Revendications

1. Composition destinée à une utilisation dans la stimulation de la vascularisation chez un sujet, la composition comprenant de la dapsone ou de la N-acétyl-dapsone ; son sel pharmaceutiquement acceptable ; un solvate ; un polymorphe ; ou une combinaison de ceux-ci.

2. Composition destinée à l'utilisation selon la revendication 1, le sujet présentant des symptômes liés à une baisse de la vascularisation.

3. Composition destinée à l'utilisation selon la revendication 2, les symptômes liés à une baisse de la vascularisation étant l'ischémie ou une maladie qui provoque l'ischémie.

4. Composition destinée à l'utilisation selon la revendication 3, l'ischémie étant l'ischémie cutanée ou l'ischémie oculaire.

5. Composition destinée à l'utilisation selon la revendication 4, l'ischémie cutanée étant la sclérodermie ou une plaie, la plaie comprenant les ulcères de décubitus, les ulcères de compression, les incisions chirurgicales, les lésions traumatiques tissulaires, les ulcères diabétiques, les brûlures, les greffes de la peau, ou une combinaison de celles-ci, et l'ischémie oculaire étant un trouble occlusif vasculaire rétinien.

6. Composition destinée à l'utilisation selon la revendication 2, les symptômes comprenant les maladies rénales chroniques non diabétiques, l'infarctus cérébral, l'infarctus du myocarde (MI), l'angine de poitrine, l'insuffisance coronaire, les complications diabétiques, les maladies du membre ischémique, l'ischémie cérébrale, les états d'inflammation vasculaire, l'artériosclérose, la nécrose avasculaire, la perte de cheveux, ou le dysfonctionnement érectile.

7. Composition pour l'utilisation dans la prévention ou le traitement des symptômes liés à une baisse de la vascularisation chez un sujet, la composition comprenant de la dapsone ou de la N-acétyl-dapsone ; son sel pharmaceutiquement acceptable ; un solvate ; un polymorphe ; ou une combinaison de ceux-ci.

8. Composition destinée à l'utilisation selon la revendication 7, les symptômes liés à une baisse de la vascularisation étant l'ischémie ou une maladie qui provoque l'ischémie.

9. Composition destinée à l'utilisation selon la revendication 8, l'ischémie étant l'ischémie cutanée ou l'ischémie oculaire.

10. Composition destinée à l'utilisation selon la revendication 9, l'ischémie cutanée étant la sclérodermie ou une plaie, la plaie comprenant les ulcères de décubitus, les ulcères de compression, les incisions chirurgicales, les lésions traumatiques tissulaires, les ulcères diabétiques, les brûlures, les greffes de la peau, ou une combinaison de celles-ci, et l'ischémie oculaire étant un trouble occlusif vasculaire rétinien.

11. Composition destinée à l'utilisation selon la revendication 7, les symptômes comprenant les maladies rénales chroniques non diabétiques, l'infarctus cérébral, l'infarctus du myocarde (MI), l'angine de poitrine, l'insuffisance coronaire, les complications diabétiques, les maladies du membre ischémique, l'ischémie cérébrale, les états d'inflammation vasculaire, l'artériosclérose, la nécrose avasculaire, la perte de cheveux, ou le dysfonctionnement érectile.

12. Composition destinée à l'utilisation selon l'une quelconque des revendications 1 à 11, la dose de dapsone ou de N-acétyl-dapsone; son sel pharmaceutiquement acceptable ; le solvate ; le polymorphe ; ou leurs combinaisons se situant dans une plage de 0,1 mg à 1 000 mg par kg du poids du sujet.

13. Composition destinée à l'utilisation selon l'une quelconque des revendications 1 à 12, la composition étant une substance pharmaceutique ou un aliment fonctionnel.

14. Dapsone ou N-acétyl-dapsone ; son sel pharmaceutiquement acceptable ; son solvate ; son polymorphe ; ou une combinaison de ceux-ci destiné-e à l'utilisation dans la prévention ou le traitement des symptômes liés à une baisse de la vascularisation chez un sujet.

15. Dapsone ou N-acétyl-dapsone ; son sel pharmaceutiquement acceptable ; son solvate ; son polymorphe ; ou une combinaison de ceux-ci destiné-e à l'utilisation dans la stimulation de la vascularisation au niveau d'un site d'un sujet faisant preuve de vascularisation réduite.
